# EUROPEAN PATENT APPLICATION

(11) **EP 3 398 510 A1**
(43) Date of publication of application: **07.11.2018**
(21) Application number: 17169584.4
(22) Date of filing: 04.05.2017
(51) Int. Cl.: A61B 5/04, A61B 5/08

(54) **SYSTEM AND METHOD FOR DYNAMIC FOCUSING ON THE HEART AND/OR LUNGS BY FREQUENCY TUNING AND ANALYSIS OF IMPEDANCE PHASE AND/OR MAGNITUDE VARIATIONS**

(71) Applicant: Koninklijke Philips N.V., 5656 AE Eindhoven (NL)
(72) Inventor: LEIJSSEN, Jacobus Josephus, 5656 AE Eindhoven (NL); DOODEMAN, Gerardus Johannes Nicolaas, 5656 AE Eindhoven (NL); Bezemer, Rick, 5656 AE Eindhoven (NL); PAULUSSEN, Igor Wilhelmus Franciscus, 5656 AE Eindhoven (NL); PEETERS, Wouter Herman, 5656 AE Eindhoven (NL); Kleijnen, Mark, 5656 AE Eindhoven (NL)
(74) Representative: de Haan, Poul Erik

(57) **Abstract**

A vital sign monitoring device (10) includes a radio frequency (RF) loop coil (12) that is resonant at both a low frequency and a high frequency that is different from and higher than the low frequency. An annular Faraday shield (18) is arranged to shield the RF loop coil. An oscillator circuit (22) is connected to the both lower and higher oscillation frequency determining RF loop. Readout electronics (24) are connected to measure an electrical response of the RF loop coil energized by the voltage source at both the low frequency and the high frequency and to: extract at least one signal component of the electrical response at the low frequency; extract at least one signal component of the electrical response at the high frequency; and generate vital sign data using both the at least one signal component of the electrical response at the low frequency and the at least one signal component of the electrical response at the high frequency.

## Description

### FIELD OF THE INVENTION

The following relates generally to the vital sign monitoring arts, cardiopulmonary monitoring arts, magnetic field monitoring arts, and related arts.

### BACKGROUND OF THE INVENTION

Unobtrusive continuous vital sign monitoring, especially of the life-critical heart rate and breathing parameters such as breathing rate and/or breath depth, is useful in a wide range of medical settings, e.g. for ambulatory patients at the general ward, patients that are sent from hospital to home, and patients receiving home care (e.g. heart failure, elderly). Several approaches have been developed over the years to measure such vital signs. For instance, there are a number of breathing rate sensors capable of determining the breathing rate on the market, such as a respiratory belt, a three dimensional (3D) accelerometer, or a photoplethysmogram (PPG) or electrocardiograph (ECG) measurement.

One way to measure the heart rate and breathing rate in an unobtrusive way is by measuring variation of electric and weak magnetic characteristics by magnetic induction in the patient's chest or arteries. This can be done using a primarily magnetic measurement field that covers the volume of the lung and/or heart of the patient. The measured magnetic and electric tissue characteristics are influenced by intrathoracic fluid movements due to cardiac cycling and breathing. These variations of the measured magnetic and electric characteristics can be used to derive breathing rate, breathing depth, and heart rate, as well as the presence of lung water (i.e., pulmonary edema).

The following discloses new and improved systems and methods to overcome these problems.

### SUMMARY OF THE INVENTION

In one disclosed aspect, a vital sign monitoring device includes a radio frequency (RF) loop coil that is resonant at both a low frequency and a high frequency that is different from and higher than the low frequency. An annular Faraday shield is arranged to shield the RF loop coil. An electronic oscillator circuit is connected to the RF loop coil and fulfils oscillation conditions at both the low frequency and the high frequency. Readout electronics are connected to measure an electrical response of the RF loop as part of the oscillator at both the low frequency and the high frequency and to: extract at least one signal component of the electrical oscillator characteristics at the low frequency; extract at least one signal component of the electrical oscillator characteristics at the high frequency; and generate vital sign data using both the at least one signal component of the electrical oscillator characteristics at the low frequency and the at least one signal component of the electrical oscillator characteristics at the high frequency. The electrical oscillator characteristics are determined by the electrical impedance expressed in magnitude and phase of the RF loop.

In another disclosed aspect, a vital sign monitoring device includes a tunable loop coil. A Faraday shield is annularly disposed about the tunable loop coil. The Faraday shield includes at least one opening in which a portion of the tunable loop coil is exposed. A voltage source is configured to supply voltage to the tunable coil. The voltage is supplied at a first frequency and second, different frequency. An impedance measurement circuit is configured to measure an impedance value of the tunable coil at each of first and second frequencies. The electrical impedance value is expressed in the magnitude and phase as indication for magnetic fields induced by the tissue under test and the dissipation of magnetic energy by conductivity of eddy currents in the tissue under test. At least one processor is programmed to: extract a first impedance magnitude and phase signal at the first frequency; extract a second impedance magnitude and phase signal at the second frequency; combine the first phase signal with the first magnitude signal to generate a first combined signal; combine the second phase signal with the second magnitude signal to generate a second combined signal; and derive at least one vital sign parameter from at least one of the first and second combined signals.

In another disclosed aspect, a method of monitoring at least one vital sign of a patient includes: with a current source, supplying current to an inner portion of a tunable coil at a first, low frequency and to an outer portion of the tunable coil at a second, high frequency to produce corresponding high and low frequency voltage signals; with a low pass filter, filtering the low frequency signal to remove high frequency signal components therefrom; with a high pass filter, filtering the high frequency signal to remove low frequency signal components therefrom; with at least one processor, combining a magnitude portion of the low frequency signal with a phase portion of the high frequency signal to generate a first combined signal; with the at least one processor, combining a magnitude portion of the high frequency signal with the phase portion of the high frequency signal to generate a second combined signal; with the at least one processor, deriving at least one of a heart rate signal from the first combined signal and a breathing rate or a breathing depth signal from the second combined signal; with a display, displaying at least one of the derived heart rate signal, breathing rate signal, and breathing depth signal.

One advantage resides in providing a unitary vital sign monitor to measure both cardiac and respiratory activity.

Another advantage resides in providing cardiac monitoring with reduced interference from respiration.

Another advantage resides in providing respiratory monitoring with reduced interference from cardiac cycling.

Another advantage resides in providing cardiac monitoring with improved signal to noise ratio (SNR).

Another advantage resides in providing respiratory monitoring with improved SNR.

Another advantage resides in providing a vital sign monitor for monitoring cardiac activity, respiratory activity, or both with improved compactness, flatness, and/or a smaller footprint when placed on the patient.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the invention.
FIG. 1A diagrammatically illustrates a coil of a vital sign monitoring device in accordance with one embodiment.
FIG. 1B diagrammatically illustrates the vital sign monitoring device in accordance with another embodiment.
FIG. 2A diagrammatically illustrates the vital sign monitoring device in accordance with another embodiment.
FIG. 2B diagrammatically illustrates the vital sign monitoring device in accordance with another embodiment.
FIG. 3A illustrates a graph of the magnitude of the RF loop impedance vs frequency of the device of FIGS. 1A and/or 1B.
FIG. 3B illustrates a graph of the phase of the RF loop impedance vs frequency of the device of FIGS. 1A and/or 1B.
FIG. 4 graphically shows signal processing operations of the device of FIGS. 1 and 2.
FIG. 5 shows a planar view of the device of FIGS. 1 and 2.
FIG. 6 diagrammatically shows an operational flow chart for operation of the device of FIGS. 1 and 2.

### DETAILED DESCRIPTION OF THE EMBODIMENTS

Magnetic fields are penetrating more deeply into the human body and are less sensitive to measurement artifacts than electrical fields. Electromagnetic fields with a dominant magnetic field are therefore more suitable for unobtrusive vital sign and lung water measurements than electromagnetic fields with a dominant electric field.

However, when monitoring variations of magnetic characteristics in the thorax with the purpose of vital sign assessment, it is hard to separate the contributions of variations of magnetic characteristics of the heart from the contributions of the lungs.

It is disclosed herein that the shape and information in the signal acquired from the human body depends strongly on the applied frequency, and that different information is contained in the impedance magnitude variations as compared with the impedance phase variations. Lower megahertz (MHz) signals are strongly influenced by the heartbeats, while higher MHz and gigahertz (GHz) signals are strongly influenced by the breathing cycles. Furthermore, the impedance phase variation preferentially contain information on the heart cycle, while the impedance magnitude variation preferentially contain information on the respiratory cycle.

Disclosed herein are new and improved vital sign monitor devices and methods operating on the basis of the foregoing observations and thereby allowing, with a single low-profile device, to dynamically focus on the heart and/or lungs by frequency tuning and analysis of phase and/or magnitude variations.

In some embodiments disclosed herein, a vital sign monitoring device provides monitoring of the heart and/or lungs with improved signal to noise ratio (SNR) by frequency tuning, using a single coil, and analysis of phase and/or magnitude variations. The vital sign monitoring device is advantageously highly robust against signal artifacts and noise sources, providing a reliable vital sign monitoring solution which is less affected by outside interferences. Furthermore, the disclosed vital sign monitoring devices can be manufactured using low cost electronics, while maintaining a high measurement reliability.

Vital sign monitoring devices are disclosed herein that employ dual frequency excitation of a single coil a planar inductive loop shielded by a Faraday shield. In this approach, the coil is simultaneously energized at two different frequencies which are resonant or near-resonant frequencies for the coil. This configuration provides deep penetration into the human body via the induced magnetic field. While the ground shield attenuates the electric field residue. A coaxial Faraday shield at the patient's side attenuates the electrical field even more. Conceptually, the lower frequency provides deeper penetration into the human body than the higher frequency; whereas, the phase shift at the higher frequency is more strongly influenced by both heart rate and respiration as compared with the phase shift at lower frequency. Advantageously, it is straightforward to employ an oscillator circuit, to compose with the coil, having an electrical oscillator that can operate at the two frequencies, and provides the frequency variation and the absorbed magnetic energy variation according to the impedance magnitude and phase variation over time.

The vital sign monitoring device may provide a combined cardiac/respiratory monitor, or may provide a cardiac-only (or respiratory-only) monitor. In the latter cases, the dual frequency usefully provides for straightforward and accurate suppression of the undesired signal (e.g., the respiratory signal in the case of a cardiac monitor).

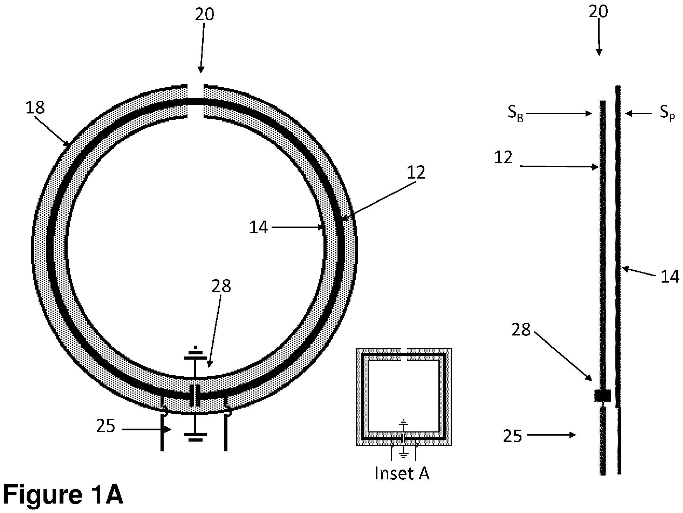

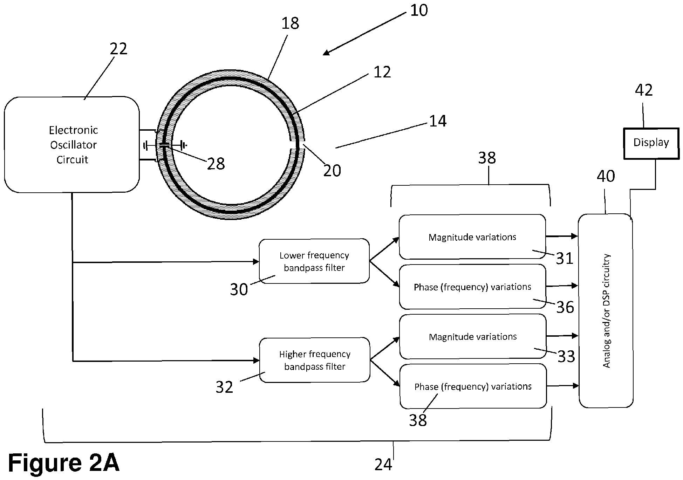

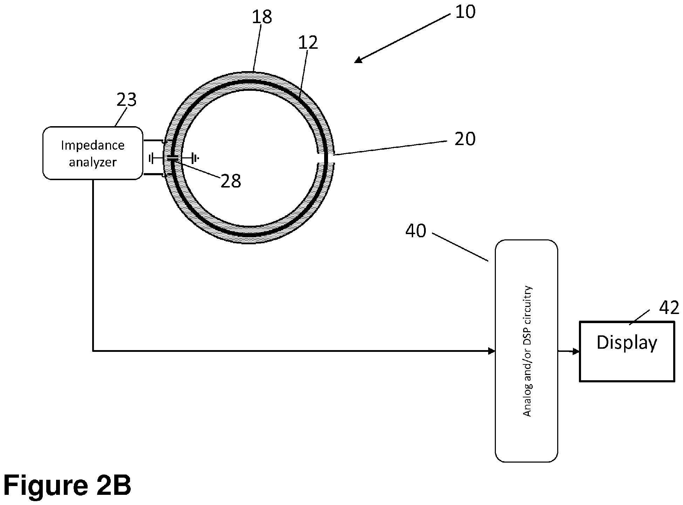

With reference to FIG. 1A, a radio frequency (RF) coil and a ground shield assembly suitable for use in an illustrative vital sign monitoring device (see FIG. 2) is shown. The assembly can be configured to measure cardiac activity of a patient, respiratory activity of a patient, or both. The assembly includes at least one radio frequency (RF) loop coil 12 and grounded shield 14, and more specifically a single RF loop coil 12 in the illustrative embodiment of FIG. 1A. The loop coil 12 is resonant at both a first, low frequency and a second, higher frequency that is different from, and higher than, the low frequency. While the illustrative loop coil 12 is circular in shape, the loop coil can have substantially any geometry, e.g. square (see "Inset A" of FIG. 1A), rectangular, hexagonal, et cetera. The loop coil 12 can optionally be tunable in that the resonant frequency can be adjusted. In some embodiments, the high frequency is within +/-10% inclusive of the third harmonic of the low frequency. In some embodiments that have been found to provide good sensitivity to cardiac and respiratory cycling, the low frequency is less than or equal to 500 MHz and the high frequency is at least one gigahertz.

In some embodiments the lower frequency would be selected from a range of 25 MHz or 100 MHz to 350 MHz; or 50 MHz or 100 MHz to 500 MHz. These operating ranges may provide an additional sensitivity of the monitoring device due to phase shifts of the eddy-currents occurring at these frequency ranges due to polarization currents.

In the frequency range of 25 MHz to 1 GHz or even 2 GHz the polarization currents (also known as displacements currents) inside a biological tissue become comparable to the conduction currents; in other words, the polarization currents become pronounced. The inventors discovered that the induction of polarization currents (or displacement currents) is beneficial for the vital signs measurement.

This may be understood in the following way, the polarization currents change the phase of a secondary magnetic field, which is beneficial for a chosen measurement configuration where the inductive loop coil 12 is brought in resonance on a capacitor 28. This configuration provides an increased sensitivity of the device to phase changes of the secondary field. Magnetic fields induced by conduction currents (which dominate at lower frequencies) are 90-degrees-phase-shifted as compared to a primary field, which leads to changes in the dissipation of a resonator (mathematically denoted as an imaginary part of the self-inductance of the loop). These changes in dissipation may be challenging to measure, and therefore often submerged in the noise level. However, magnetic fields induced by polarization currents (or displacement currents, which start dominating in 25 MHz to 500 MHz or 1 GHz frequency range), are in-phase with the primary field, which leads to a frequency shift of the resonator. Frequency shifts can be measured more precisely, and thus providing the vital sign monitoring device with improved sensitivity compared to a sensor that depends on primarily conduction currents.

Further, a relative strength of the polarization currents (or displacement currents) as compared to the conduction currents differs for different tissues in our proposed frequency range. A selection of frequencies in the range of 25 MHz -100 MHz allow distinguishing fat from muscle and blood, while frequencies in the range from 100 MHz-500 MHz allow distinguishing muscle from blood.

Therefore, 25 MHz -500 MHz in the range wherein the vital signs measurement implementation becomes sensitive to spatial and temporal changes in the tissue type that is located in the probed volume of the loop. A beating heart can be seen as a periodically changing blood volume and muscle volume. A breathing lung can be seen as a periodically changing air volume and muscle volume. Thus, by operating in the proposed frequency range, the polarization currents (or displacement currents) are pronounced, thereby enhancing the signal, and allowing the device to be sensitive to spatial and temporal variations of the tissue type.

The lower limit of the frequency ranges disclosed herein might beneficially selected to be 50 MHz (instead of 25 MHz). This frequency limit further provides an improved sensitivity due to a skin effect causing the eddy currents occurring closer to the loop coil, which increases the signal strength. Further, the skin effect causes in-phase eddy currents, for which the discussed vital sign monitoring device shows a further increase in sensitivity (within the detection space of the loop).

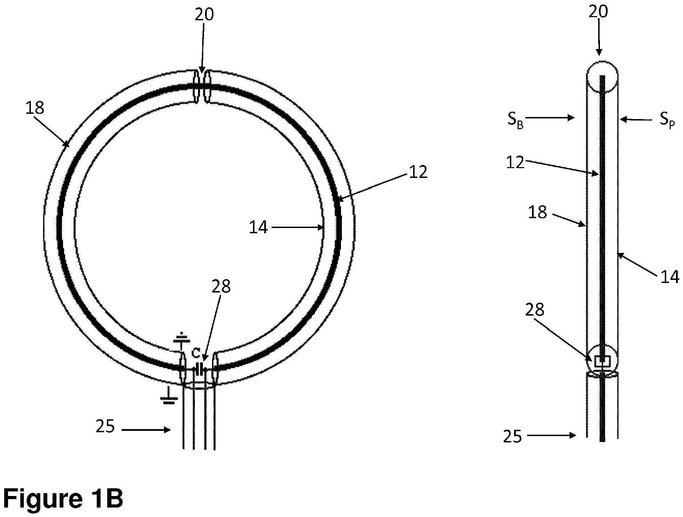

As shown in FIG. 1B, and with continuing reference to FIG. 1A, a Faraday shield 18 is arranged to overlap the loop coil 12. The Faraday shield 18 shields the loop coil 12 from undesired radio frequency interference (RFI) from outside the coil 12. As seen in the side view of FIG. 1B, the RF coil shield assembly is low-profile, i.e. substantially flat, and has a patient side S_{P} and a back side S_{B} - the patient side S_{P} faces the chest or other probed body part of the patient, while the back side S_{B} faces away from the patient's chest. Thus, the loop coil 12 is facing and proximate to the patient's chest, while the grounded shield 14 is located distal from the patient and is thereby positioned to shield the loop coil 12 from external RFI. The Faraday shield 18 overlaps the loop coil 12. In the illustrative example, the Faraday shield 18 is annular to surround a round loop coil 12. The ground shield 14 and the Faraday shield 18 includes at least one opening 20 to suppress eddy currents in the shield 14 or 18.

Referring now to FIG. 2A, a variant RF coil/Faraday shield assembly includes the round flat coil 12 and the Faraday shield 18. An oscillator circuit 22 is connected to the loop coil 12. The oscillator circuit 22 is configured to oscillate with the loop coil at two or more frequencies (i.e., both the low frequency and the high frequency).

A vital sign monitoring device 10 includes the RF coil 12 and ground shield 14 (or Faraday shield 18), and also includes readout electronics 24 connected to the loop coil 12 and the ground shield 14 or the Faraday shield 18 by a transmission line 25. The readout electronics 24 are programmed to measure an electrical response of the loop coil 12 energized by the oscillator circuit 22 at both the low frequency and the high frequency. In some examples, the readout electronics 24 are programmed to separately measure magnitude and phase of the eddy current-induced magnetic fields in the body under test by the mutual inductance to loop coil 12, and extract the eddy current path length and electrical conductivity in the body of the coil at each of the first and second frequencies. The frequencies can be adjusted based on the measured eddy current-induced magnetic field and/or conductivity.

In some embodiments, the readout electronics 24 include the capacitor 28 operably disposed on a portion of the loop coil 12, the capacitor 28 coupled to the loop together form a resonant circuit. The electronic oscillator circuit 22 outputs a lower frequency impedance signal and a higher frequency impedance signal, which are processed by the readout electronics 24 as described next.

As disclosed herein, the cardiac and/or respiratory signals are effectively segregated out by frequency filtering. Accordingly, the readout electronics 24 include a lower frequency band pass filter 30 that passes the lower frequency signal from which impedance magnitude variations 31 and phase variations 36 are obtained. Likewise, a higher frequency band pass filter 32 passes the higher frequency signal from which loop impedance magnitude variations 33 and phase variations 38 are obtained, as described in more detail below. As further disclosed herein, analysis of the phase variation of the loop impedance variations provides additional or alternative information for segregating out the cardiac and/or respiratory signals.

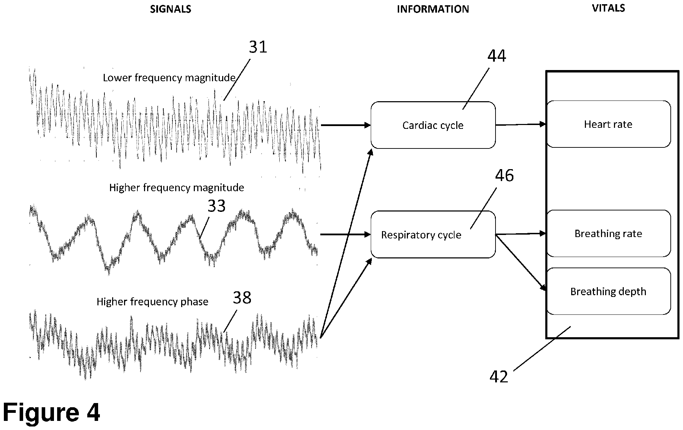

While FIG. 2A illustrates a generic embodiment, in specific implementations it is contemplated to omit generation of one or more of the frequency-filtered RF loop impedance magnitude and/or phase signals 31, 33, 36, 38. By way of illustration, the embodiment described later herein with reference to FIG. 4 does not utilize the low frequency phase shift signal 36 in generating vital sign data, and hence this signal 36 may optionally not be generated in the embodiment of FIG. 4. The derived frequency-filtered magnitude and/or phase signals 31, 33, 36, 38 are further processed by analog or digital circuitry 40 to generate vital sign signals, which are shown on a display 42.

The electronics 24, 40 may include analog components, typical computer and/or signal processing electronic components. In general, the various electronic signal processing components 22, 30, 32, 40 can be implemented as analog electronics such as analog filters, operational amplifier (op-amp) circuits, and/or so forth, and/or as digital signal processing (DSP) components (e.g. dedicated DSP chips, computer processing performed by the microprocessor of a patient monitor, or so forth) after analog-to-digital (A/D) conversion using A/D converters of the return loss or other signal output from the coil 12.

In general, the vital sign monitoring device 10 can be placed on a thorax of the patient whose vital sign(s) are to be monitored. The loop coil 12 is part of oscillator circuit 22 and oscillates at the (low) resonance frequency (e.g., 350 MHz at the inner portion of the coil 12) and the (high) resonance frequency (e.g., 1400 MHz at the outer portion of the coil) for optimal sensitivity to magnetic impedance modulations caused by cycling of the heart and lungs.

The approach of FIG. 2A employs the oscillator 22 whose output is analyzed by electronics 24 to generate the various impedance magnitude and phase variations 31, 33, 36, 38 (or, in some embodiments, some sub-set of these). This provides low cost and low power hardware.

With reference to FIG. 2B, in some other contemplated embodiments, alternative readout electronics are employed. By way of non-limiting illustration, in the embodiment of FIG. 2B, an impedance measurement circuit or analyzer 23 is directly coupled to the resonant circuit, without the oscillator 22 of FIG. 2A, and is configured to measure an impedance value of the coil loop 12 at each of the first and second frequencies. Compared with the approach of FIG. 2A employing the oscillator 22, the approach of FIG. 2B is expected to be more expensive and less energy-efficient.

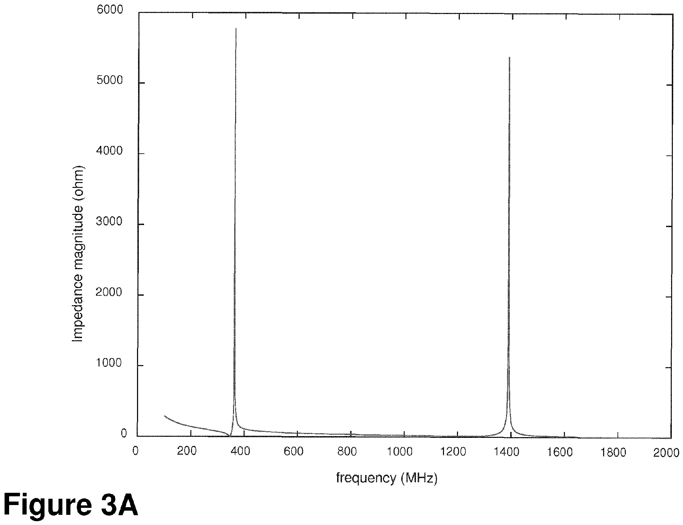

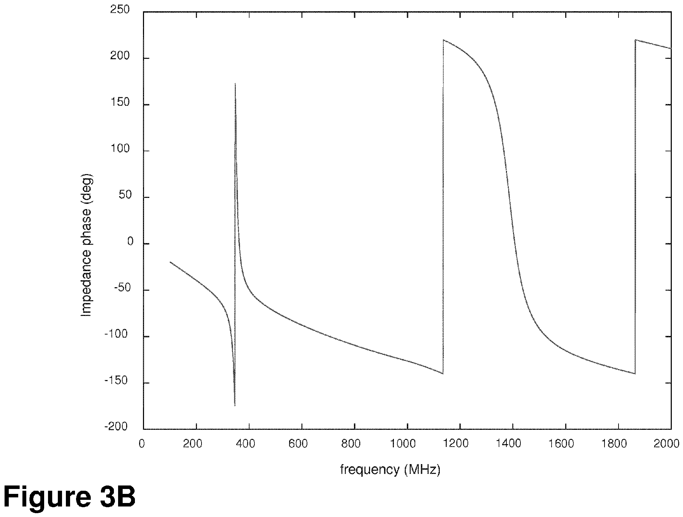

FIG. 3A and FIG. 3B illustrate that maximum sensitivity is obtained at or proximate to the coil resonances. Variations in the magnetic impedance in the thorax, caused by fluid and air shifts caused by heartbeats and breathing, affects the impedance of the coil 12, and thereby the oscillator frequency and the negative impedance of the oscillator circuit. The Faraday shield 18 prevents interference from outside the device 10, and the opening 20 prevents eddy currents in the shield.

In one illustrative signal processing approach shown in FIG. 4, the analog and/or DSP circuitry 40 generates both cardiac and respiratory vital signs by processing the lower frequency magnitude signal 31, the higher frequency magnitude signal 33, and the high frequency phase shift signal 38. More specifically, in this illustrative embodiment a cardiac signal 44 is generated by combining the lower frequency magnitude signal 31 and the higher frequency phase variation signal 38; while, a respiratory signal 46 is generated by combining the higher frequency magnitude signal 33 and the higher frequency phase shift signal 38. At least one vital sign parameter is derived from the cardiac and respiratory signals 44, 46. For example, a heart rate signal can be derived from the cardiac signal 44 as the principle periodicity, e.g. extracted by a Fourier transform, determining an average peak-to-peak time interval, or so forth. Similarly, a breathing rate signal can be derived as the principle frequency of the respiratory signal 46. A breathing depth signal can also be derived from the respiratory signal 46, e.g. as the magnitude of, or a peak-to-valley metric of, the respiratory signal 46. The heart rate signal, the breathing signal, and/or the breathing depth signal can then be transmitted by the processor 40 to the display 42 for display thereon, or transmitted to a computer system (e.g., a phone, watch, or PC) to a user.

In some actually constructed embodiments, the coil was operated simultaneously with two resonance frequencies, 350 MHz and 1100 MHz. Other frequency pairs related approximately 1:3 in frequency are also contemplated as easily designed resonant coils. An approximate third harmonic that differs from the exact third harmonic by 10% or less is expected to be advantageous for simplifying design of the driving circuitry and resonant coil 12. The Faraday shield 18 ensures that the magnetic field portion of the electromagnetic field dominates the behavior of the resonant coil 12. The two frequencies can be selected from the range of 25 MHz to 1 GHz or even 2 GHz. An improved device 10 sensitivity may be achieved by selecting the first frequency from 25 MHz (or 50 MHz) to about 100 MHz, preferably about 80-90 MHz; and by selecting the second frequency from above 100 MHz to 500 MHz (or even to 1 GHz).

While the illustrative example of FIG. 4 outputs both cardiac and respiratory signals, in other embodiments the vital sign monitoring device may be constructed to output only cardiac data, or only respiratory data. The dual-frequency approach disclosed herein is still beneficial in these embodiments as it permits suppression of the "other" signal, i.e. suppression of the heartbeat if breathing is measured; or conversely, suppression of the breathing if the heartbeat is measured.

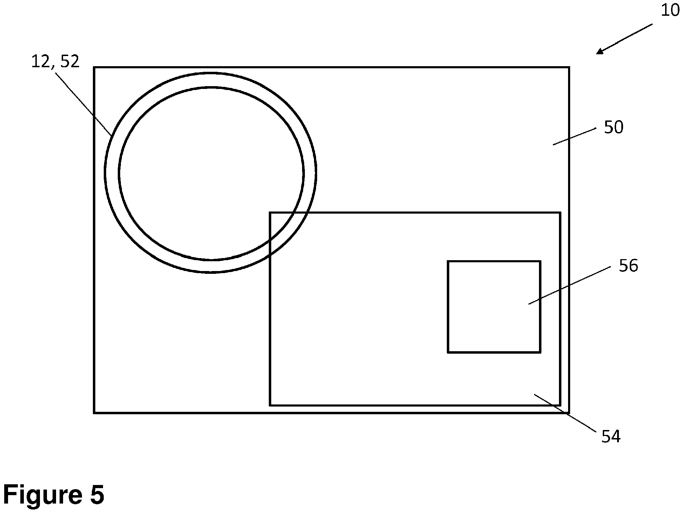

Referring now to FIG. 5, an illustrative physical construction embodiment of the device 10 is diagrammatically shown. As shown in FIG. 5, the vital sign monitoring device 10 further includes a printed circuit board (PCB) 50. The coil 12 comprises a printed circuit 52 disposed on the PCB 50. The readout electronics 24 include at least one of an electric circuit 54 and a microchip 56 disposed on the PCB 50. In actually constructed embodiments, a low-profile (i.e. substantially flat) vital sign monitoring device was constructed by employing a small printed circuit board (PCB) on which the coil 12 is printed with an inner layer of the PCB with bottom and top layers of the PCB serving as a ground plated Faraday shield 18, with components such as the oscillator circuit soldered to the PCB with PCB traces serving for interconnects.

In this example, the device 10 is a unitary planar device. It is assumed in this embodiment that the output of the device 10 is sent to a patient monitor, smart phone, or other separate device (not shown) for display and/or vital sign data storage, e.g. via a wireless transmission (in which case the electronics 24 include a Bluetooth or other wireless transmitter) or a wired connection (e.g., a micro-USB port with soldered connections to the PCB 40). Additionally or alternatively, it is contemplated to include an on-board display (not shown), e.g. an LCD readout display, mounted on the PCB 40. The device 10, as shown in FIG. 5, is very flat, and can be constructed at low cost, and can be embedded in a hand-held system for screening and triage, or in a (disposable) wearable device or patch for prolonged monitoring of (fetal) vital signs and/or lung conditions. When the device 10 is embedded in, for example, a patch, the device can be tuned towards high sensitivity for the heart (combine low frequency amplitude modulations and high frequency phase shift modulations), towards high sensitivity for the lungs (combine high frequency amplitude and phase shift modulations), or a combination of both.

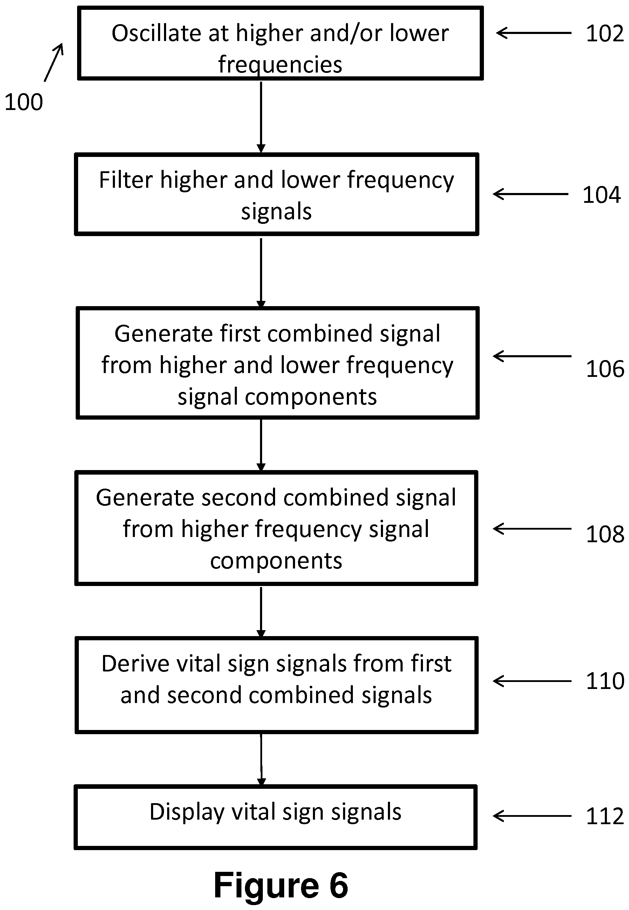

With reference to FIG. 6, operation of the device 10 is diagrammatically flowcharted as a method 100. At 102, the voltage source 22 supplies voltage to the inner and outer portions of the coil 12 at the corresponding low and high frequencies to produce a high frequency signal and a low frequency signal. At 104, the low and high pass filters 30 and 32 filter the corresponding high and low frequency signals. At 106, the processor 40 combines an amplitude portion of the low frequency signal with a phase portion of the high frequency signal to generate a first combined signal. At 108, the processor 40 combines an amplitude portion of the high frequency signal with the phase portion of the high frequency signal to generate a second combined signal. At 110, the processor 40 derives a heart rate signal from the first combined signal and/or a respiration rate or depth signal from the second combined signal. At 112, the heart rate signal, the respiration rate signal, and/or the respiration depth signal are displayed on the display 42.

By way of some non-limiting illustrative clinical applications, the device 10 is valuable for ambulatory monitoring of cardiac (e.g., post-surgery, heart failure, hypertension, elderly) and respiratory (e.g., COPD, asthma, pneumonia) patients in the hospital, from hospital to home, and at home. The device 10 could also be potentially valuable for unobtrusive fetal monitoring or check-ups, making use of low-level magnetic fields rather than electrical fields or ultrasound.

The disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

In one of the embodiments the processor 38 is further programmed to: derive a heart rate signal from the first combined signal; and derive at least one of a breathing rate signal and a breathing depth signal from the second combined signal.

The device 10 according to some of the embodiments may include the capacitor 28 operably disposed on a portion of the tunable coil, the capacitor being configured to measure an impedance value of the tunable coil at each of first and second frequencies. It may also further comprise: a low pass filter 30 configured to filter the second frequency from the first amplitude signal; and a high pass filter 32 configured to filter the first frequency from the second amplitude signal and the phase shift signal.

In yet another embodiment a method 100 of monitoring at least one vital sign of a patient is presented, the method comprising: with an oscillator 22 with frequency determining tunable coil 12 and at least one of a ground shield 14 and a Faraday shield 18, determining a first, low frequency by capacitor 28 and a second, high frequency by an outer portion of gap 20 to produce corresponding high and low frequency signals; with a low pass filter 30, filtering the low frequency signal to remove high frequency signal components therefrom; with a high pass filter 32, filtering the high frequency signal to remove low frequency signal components therefrom; with at least one processor 40, combining an amplitude portion of the low frequency signal with a phase portion of the high frequency signal to generate a first combined signal; with the at least one processor 40, combining an amplitude portion of the high frequency signal with the phase portion of the high frequency signal to generate a second combined signal; and
with the at least one processor (40), deriving at least one of a heart rate signal from the first combined signal and a breathing rate or a breathing depth signal from the second combined signal.

The method 100 may further include with a display (42), displaying at least one of the derived heart rate signal, breathing rate signal, and breathing depth signal.

## Claims

1. A vital sign monitoring device (10), comprising:
a radio frequency (RF) loop coil (12) that is resonant at both a low frequency and a high frequency that is different from and higher than the low frequency;
an annular Faraday shield (18) arranged to shield the RF loop coil;
an electronic oscillator circuit (22) connected to the RF loop coil to form an oscillator at both the low frequency and the high frequency;
readout electronics (24) connected to measure an electrical response of the RF loop coil energized by the voltage source at both the low frequency and the high frequency and to:
extract at least one signal component of the electrical oscillator response at the low frequency;
extract at least one signal component of the electrical oscillator response at the high frequency; and
generate vital sign data using both the at least one signal component of the electrical response at the low frequency and the at least one signal component of the electrical response at the high frequency.

2. The device (10) according to claim 1, wherein the at least one signal component of the electrical response at the low frequency includes a loop impedance magnitude signal component at the low frequency and the vital sign data includes a heart rate derived from at least the impedance magnitude signal component at the low frequency.

3. The device (10) according to claim 2, wherein the at least one signal component of the electrical response at the high frequency includes a loop impedance phase shift signal component at the high frequency and the heart rate is derived from both the magnitude signal component at the low frequency and a signal produced by high pass filtering the phase shift signal component at the high frequency.

4. The device (10) according to any one of claims 1-3, wherein the vital sign data includes a respiration rate derived from the at least one signal component of the electrical response at the high frequency.

5. The device (10) according to claim 4, wherein the at least one signal component of the electrical response at the high frequency includes a magnitude signal component at the high frequency and the respiration rate is derived from at least the magnitude signal component at the high frequency.

6. The device (10) according to either one of claims 4 and 5, wherein the at least one signal component of the electrical response at the high frequency includes a phase shift signal component at the high frequency and the respiration rate is derived by operations including at least low pass filtering the phase shift signal component at the high frequency.

7. The device (10) according to any one of claims 1-6, wherein the readout electronics (24) are connected to measure the electrical response comprising the impedance of the RF loop coil (12) energized by the impedance analyzer (23) at both the low frequency and the high frequency.

8. The device (10) according to claim 7, wherein the readout electronics (24) are programmed to measure the electrical response by at least one of measuring eddy current-induced magnetic fields in the RF loop coil (12) and eddy-current induced conductivity in the RF loop coil.

9. The device (10) according to any one of claims 1-8, further comprising a printed circuit board (PCB) (50) wherein the RF loop coil (12) comprises a printed circuit (52) disposed on the PCB.

10. The device (10) according to claim 9, wherein the readout electronics (24) comprise at least one of an electric circuit (54) and a microchip (56) disposed on the PCB (50) and the vital sign monitoring device is a unitary planar device.

11. The device (10) according to any one of claims 1-10, wherein the current source (22) comprises an electronic oscillator circuit.

12. The device (10) according to any one of claims 1-11, further including a display (42) configured to display the derived at least one vital sign parameter.

13. The device (10) according to any one of claims 1-12, wherein the low frequency is less than or equal to 500 MHz and the high frequency is at least one gigahertz.

14. The device (10) according to any one of claims 1-13, wherein the readout electronics (24) include a capacitor (28) operably disposed on a portion of the RF coil (12), the capacitor being configured to measure an impedance value of the RF coil at each of first and second frequencies.

15. A vital sign monitoring device (10), comprising:
a tunable loop coil (12);
at least one of a ground shield (14) and a Faraday shield (18) annularly disposed about the tunable loop coil, the at least one of the ground shield and the Faraday shield including at least one opening (20) in which a portion of the tunable loop coil is exposed;
an electronic oscillator circuit (22) configured to oscillate with the tunable coil as frequency determined element at a first frequency and second, different frequency; and
readout electronics (24) including at least one processor (40) programmed to:
generate a first amplitude signal from the first frequency;
generate a second amplitude signal and a phase shift signal at the second frequency;
combine the phase shift signal with the first amplitude signal to generate a first combined signal;
combine the phase shift signal with the second amplitude signal to generate a second combined signal; and
derive at least one vital sign parameter from at least one of the first and second combined signals.

16. The device (10) according to claim 15, wherein the at least one processor (38) is further programmed to:
derive a heart rate signal from the first combined signal; and
derive at least one of a breathing rate signal and a breathing depth signal from the second combined signal.
